# EUROPEAN PATENT SPECIFICATION

(11) **EP 3 620 107 B1**
(45) Date of publication and mention of the grant of the patent: **04.11.2026**
(21) Application number: 19194782.9
(22) Date of filing: 30.08.2019
(51) Int. Cl.: A61B 5/00, A61B 5/332

(54) **SCALING PHYSIOLOGICAL SIGNALS**
SKALIERUNG PHYSIOLOGISCHER SIGNALE
MISE À L'ÉCHELLE DE SIGNAUX PHYSIOLOGIQUES

(30) Priority: 10.09.2018 US 201862729288 P; 28.08.2019 IN 201914034728
(43) Date of publication of application: 11.03.2020
(73) Proprietor: Apple Inc., Cupertino CA 95014 (US)
(72) Inventor: Mollazadeh, Mohsen, Cupertino, CA 95014 (US); Wahabi, Saeid, Cupertino, CA 95014 (US); Brittain, Roxanne, Cupertino, CA 95014 (US)
(74) Representative: Barton, Russell Glen

(56) References cited:
- EP-A1- 1 552 786
- JP-A- H05 154 118
- US-A- 5 493 515

## Description

### CROSS-REFERENCE TO RELATED APPLICATIONS

This application claims the benefit of U.S. Provisional Application No. 62/729,288, filed September 10, 2018.

### FIELD

This relates generally to systems and methods of processing physiological signals, and more particularly, to scaling physiological signals based on characteristics of the signals.

### BACKGROUND

Electrocardiogram (ECG) waveforms can be generated based on the electrical activity of the heart during each heartbeat. The waveforms can be recorded from multiple electrical leads attached to various areas of a patient. For example, a 12-lead ECG system with a group of six measurement electrodes that can be placed across the patient's chest, and a group of six measurement electrodes that can be attached to the patient's limbs. The measurement electrodes for ECG data acquisition can include a conducting or electrolytic gel (e.g., Ag/AgCl gel) to provide a continuous, electrically-conductive path between the skin and the electrodes. Such "wet" electrodes can reduce the impedance at the electrode-skin interface, thereby facilitating the acquisition of a low-noise ECG signal. All of the measurement electrodes can be connected to a device where signals from the measurement electrodes can be transmitted for storage, processing, and/or displaying. Devices with numerous "wet" electrodes coupled to the user's chest and limbs are invasive, may be difficult to operate for a layperson, and the result ECG waveform may be difficult to interpret. As a result, ECG measurements, analysis and heart rhythm classification may be limited the usage of ECG devices to a medical setting or by medical professionals.

Due to physiology of a user and other potential environmental factors, the amplitude (and thereby dynamic range) of ECG signal can vary. For example, factors such as placement of the electrodes against the user's skin, the heart orientation relative to the electrodes, the physical activity of the user, and other environmental factors can affect the amplitude or dynamic range of an ECG signal. In particular, the ECG signal in certain users can have a small amplitude or dynamic range, which may be difficult to interpret or which may be misinterpreted as an irregular ECG signal (e.g., the signal may appear as a "flatline" signal). Further background information can be found from EP 1552786 A1 which is directed to a graph display processing device and method that facilitates easy viewing of data with periodicity, US 5493515 A which is directed to a twice scaled waveform display apparatus which combines a twice scaled factor indication with an autoscaled waveform display, and JP H05154118 A which is directed to a portable electrocardiograph with a means for superposing a heart rate/trend graph and an event waveform so as to become two upper and lower stages on a screen of a liquid crystal device and displaying them simultaneously.

### SUMMARY

The invention is defined by the appended set of claims. This disclosure relates to devices and methods of using a mobile or wearable device for the processing of physiological signals (e.g., ECG signals) for display on the mobile or wearable device. The mobile or wearable device can comprise one or more measurement electrodes, one or more reference electrodes, and processing circuitry coupled to the electrodes. In some examples, one or more digital signal processing circuits can determine the dynamic range of the ECG signal within a given time interval (e.g., a difference between the smallest voltage level and the largest voltage level in a given time interval) and determine whether the ECG signal should be scaled (e.g., amplified, stretched, or otherwise modified) based on the dynamic range of the ECG signal. The one or more digital signal processing circuits can determine a scaling factor and apply the scaling factor to the ECG signal. The scaled ECG signal can be displayed on the display of the mobile or wearable device. In some examples, the scaling can be performed in real-time. In some examples, the scaling can be applied to ECG signals using a scaling factor determined based on the analysis and processing of an ECG signal from a previous time period (e.g., based on the previous time interval, more than one previous time interval, or any other suitable lookback time period).

### BRIEF DESCRIPTION OF THE DRAWINGS

FIGs. 1A-1C illustrate example systems in which physiological signal analysis and processing according to examples of the disclosure may be implemented.
FIGs. 4A-4B illustrate example displays of physiological signals according to examples of the disclosure.
FIGs. 5A-5B illustrate example raw physiological signal measurements according to examples of the disclosure.
FIGs. 6A-6B illustrates an example display of the physiological signal according to examples of the disclosure.
FIG. 7 illustrates an example raw physiological signal according to examples of the disclosure.
FIGs. 8A-8B illustrates an example display of the physiological signal according to examples of the disclosure.
FIGs. 9A-9C illustrate example scaling factor functions according to examples of the disclosure.

### DETAILED DESCRIPTION

In the following description of examples, reference is made to the accompanying drawings which form a part hereof, and in which it is shown by way of illustration specific examples that can be practiced. It is to be understood that other examples can be used and structural changes can be made without departing from the scope of the disclosed examples.

This relates to devices and methods of using a mobile or wearable device (or other dedicated device) for the processing of physiological signals (e.g., ECG signals) for display on the mobile or wearable device (or other wearable or non-wearable electronic device). The mobile or wearable device can comprise one or more measurement electrodes, one or more reference electrodes, and processing circuitry coupled to the electrodes. In some examples, one or more digital signal processing circuits can determine the dynamic range of the ECG signal within a given time interval (e.g., a difference between the smallest voltage level and the largest voltage level in a given time interval) and determine whether the ECG signal should be scaled (e.g., amplified, stretched, or otherwise modified) based on the dynamic range of the ECG signal. The one or more digital signal processing circuits can determine a scaling factor and apply the scaling factor to the ECG signal. The scaled ECG signal can be displayed on the display of the mobile or wearable device. In some examples, the scaling can be performed in real-time. In some examples, the scaling can be applied to ECG signals using a scaling factor determined based on the analysis and processing of an ECG signal from a previous time period (e.g., based on the previous time interval, more than one previous time interval, or any other suitable lookback time period).

It is understood that the scaling of the physiological signal described herein may be for the purpose of display (e.g., to normalize or increase the displayed amplitude of a small physiological signal for improved viewing and/or improved use of the user interface area, or to attenuate a large physiological signal to avoid clipping, etc.), but that the unscaled physiological signal may be stored and/or analyzed. In some examples, the scaled physiological signal for the purpose of display may also be stored and/or analyzed. Analysis of the physiological signal may include identify one or more physiological signal features (e.g., timing of certain waves, intervals, complexes of the ECG waveform, etc.) or conditions (e.g., heart rate, arrhythmias, atrial fibrillation, changes due to medications or surgery, function of pacemakers, heart size, etc.). Storage of the physiological signal may facilitate later review of the physiological signal (e.g., by a user or the user's healthcare provider should the user wish to share this information). To this end, it may be desirable for the reviewing person to know the unmodified scale of the physiological signal (by storing the unscaled physiological signal).

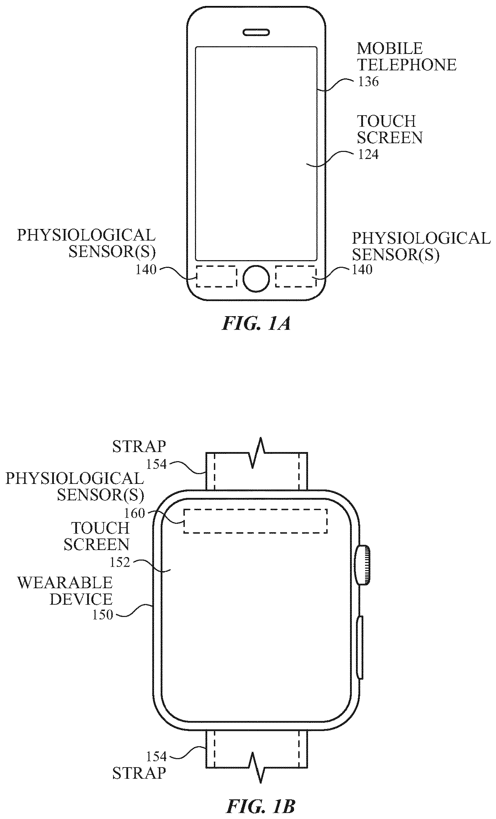

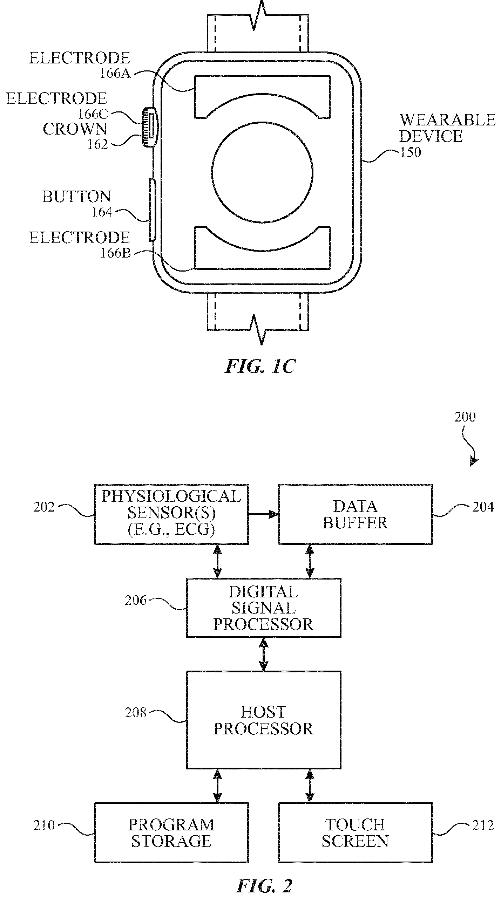

FIGs. 1A-1C illustrate example systems in which physiological signal analysis and processing according to examples of the disclosure may be implemented. FIG. 1A illustrates an example mobile telephone 136 (e.g., a smartphone) that includes an integrated touch screen 124 and one or more physiological sensor(s) 140. For example, the one or more physiological sensors can include at least one ECG sensing system including one or more measurement electrodes, one or more reference electrodes, and processing circuitry coupled to the electrodes. FIG. 1B illustrates an example wearable device 150 (e.g., a watch) that includes an integrated touch screen 152 and physiological sensor(s) 160 (e.g., an ECG sensing system including one or more measurement electrodes, one or more reference electrodes, and processing circuitry coupled to the electrodes). Wearable device 150 can be attached to a user using a strap 154 or any other suitable fastener. FIG. 1C illustrates an example of the back side of wearable device 150 that includes electrodes 166A-C of physiological sensor 160. Physiological sensor 160 can include electrode 166C implemented in crown 162 of wearable device 150, electrode implemented in button 164 of wearable device 150 (not shown), electrode 166A on the back side of wearable device 150 and/or electrode 166B on the backside of wearable device 150. In some examples, the physiological sensor 160 can include a measurement electrode (e.g., electrode 166C in crown 162), a first reference electrode (e.g., electrode 166A on the backside of wearable device 150) and a second, ground reference electrode (electrode 166B on the backside of wearable device 150). In some examples, the physiological sensor 160 can include more than one measurement electrode and more than two reference electrodes. It is understood that the above physiological sensor(s) can be implemented in other wearable and non-wearable devices, including dedicated devices for the acquisition and/or processing of physiological signals (e.g., ECG signals) for analysis and processing. It is understood that although mobile device 136 and wearable device 150 include a touch screen, the display of physiological signals described herein can be performed on a touch-sensitive or non-touch-sensitive display of the device including physiological sensor(s) 140, 160, of a separate device or a standalone display. Additionally it is understood that although the disclosure herein primarily focuses on ECG signals, the disclosure can also applicable to other physiological signals.

In some examples, the electrodes of physiological sensors 140, 160 can be dry electrodes which can be measurement electrodes configured to contact a skin surface and capable of obtaining an accurate signal without the use of a conducting or electrolytic gel. In some variations, one or more reference electrodes may be located on a wrist-worn device, such as a bracelet, wrist band, or watch, such that the reference electrodes can contact the skin in the wrist region, while one or more measurement electrodes can be configured to contact a second, different skin region. In some examples, the measurement electrode(s) can be located on a separate component from the reference electrode(s). In some examples, some or all of the measurement electrode(s) can be located on a wrist or finger cuff, a fingertip cover, a second wrist-worn device, a region of the wrist-worn device that can be different from the location of the reference electrode(s), and the like. In some examples, one or more electrodes (e.g., reference electrode or measurement electrode) may be integrated with an input mechanism of the device (e.g., a rotatable input device, a depressible input device, or a depressible and rotatable input device, for example), as shown in FIG. 1C. One or more electrical signals measured by the one or more measurement electrodes can be analyzed and processed as described in more detail herein.

FIG. 2 illustrates a block diagram of an example computing system 200 that illustrates one implementation of physiological signal processing according to examples of the disclosure. Computing system 200 can be included in, for example, mobile telephone 136, wearable device 150 or any mobile or non-mobile, wearable or non-wearable computing device for physiological signal analysis and/or display. Computing system 200 can include one or more physiological sensors 202 (e.g., ECG sensors) including one or more electrodes to measure electrical signals (e.g., ECG signals) from a person contacting the ECG sensor(s) electrodes, data buffer 204 (or other volatile or non-volatile memory or storage) to store temporarily (or permanently) the physiological signals from the physiological sensors 202, digital signal processor (DSP) 206 to analyze and process the physiological signals, host processor 208, program storage 210, and touch screen 212 to perform display operations (e.g., to display real time ECG signals). In some examples, touch screen 212 may be replaced by a non-touch sensitive display.

Host processor 208 can be connected to program storage 210 to execute instructions stored in program storage 210 (e.g., a non-transitory computer-readable storage medium). Host processor 208 can, for example, provide control and data signals to generate a display image on touch screen 212, such as a display image of a user interface (UI). Host processor 206 can also receive outputs from DSP 206 (e.g., scaled or unscaled ECG signal) and performing actions based on the outputs (e.g., display the scaled or unscaled ECG signal, play a sound, provide haptic feedback, etc.). Host processor 208 can also receive outputs (touch input) from touch screen 212 (or a touch controller, not-shown). The touch input can be used by computer programs stored in program storage 210 to perform actions that can include, but are not limited to, moving an object such as a cursor or pointer, scrolling or panning, adjusting control settings, opening a file or document, viewing a menu, making a selection, executing instructions, operating a peripheral device connected to the host device, answering a telephone call, placing a telephone call, terminating a telephone call, changing the volume or audio settings, storing information related to telephone communications such as addresses, frequently dialed numbers, received calls, missed calls, logging onto a computer or a computer network, permitting authorized individuals access to restricted areas of the computer or computer network, loading a user profile associated with a user's preferred arrangement of the computer desktop, permitting access to web content, launching a particular program, encrypting or decoding a message, and/or the like. Host processor 220 can also perform additional functions that may not be related to touch processing and display.

Note that one or more of the functions described herein, including the analysis and processing of physiological signals, can be performed by firmware stored in memory (e.g., in DSP 206) and executed by one or more processors (in DSP 206), or stored in program storage 210 and executed by host processor 208. The firmware can also be stored and/or transported within any non-transitory computer-readable storage medium for use by or in connection with an instruction execution system, apparatus, or device, such as a computer-based system, processor-containing system, or other system that can fetch the instructions from the instruction execution system, apparatus, or device and execute the instructions. In the context of this document, a "non-transitory computer-readable storage medium" can be any medium (excluding signals) that can contain or store the program for use by or in connection with the instruction execution system, apparatus, or device. The computer-readable storage medium can include, but is not limited to, an electronic, magnetic, optical, electromagnetic, infrared, or semiconductor system, apparatus or device, a portable computer diskette (magnetic), a random access memory (RAM) (magnetic), a read-only memory (ROM) (magnetic), an erasable programmable read-only memory (EPROM) (magnetic), a portable optical disc such a CD, CD-R, CD-RW, DVD, DVD-R, or DVD-RW, or flash memory such as compact flash cards, secured digital cards, USB memory devices, memory sticks, and the like.

The firmware can also be propagated within any transport medium for use by or in connection with an instruction execution system, apparatus, or device, such as a computer-based system, processor-containing system, or other system that can fetch the instructions from the instruction execution system, apparatus, or device and execute the instructions. In the context of this document, a "transport medium" can be any medium that can communicate, propagate or transport the program for use by or in connection with the instruction execution system, apparatus, or device. The transport medium can include, but is not limited to, an electronic, magnetic, optical, electromagnetic or infrared wired or wireless propagation medium.

It is to be understood that the computing system 200 is not limited to the components and configuration of FIG. 2, but can include other or additional components (or omit components) in multiple configurations according to various examples. For example, an analog-to-digital converter (ADC) may be added between physiological sensor 202 and DSP 206 to convert the signals to the analog domain or touchscreen 212 can be omitted and the ECG signal or other information from the analysis and processing can be relayed to another device (e.g., a tablet, laptop, smartphone, computer, server, etc.) via wired or wireless connection that can include a display or other feedback mechanism for outputting a visual representation of the data or other notifications or information. Additionally, the components of computing system 200 can be included within a single device, or can be distributed between multiple devices.

Returning back to physiological sensor(s) 202, the mobile or wearable device (or other device) may comprise a plurality of measurement electrodes and one or more reference electrodes. Physiological sensors 202 can be in communication with DSP 206 to acquire physiological signals and transmit the signals to DSP 206. In some examples, the physiological signals can be acquired by data buffer 204 and the DSP 206 can acquire a buffered sample of the physiological waveform (e.g., 0.5 second sample, 3 second sample, 5 second sample, 10 second sample, 30 second sample, 60 second sample). In some examples, data buffer 204 can be implemented as part of DSP 206. It should be understood that although a DSP is described, other processing circuits could be used to implement the analysis and processing described herein including a microprocessor, central processing unit (CPU), programmable logic device (PLD), and/or the like.

Although the examples and applications of analysis and processing devices and methods are described in the context of generating a complete ECG waveform, it should be understood that the same or similar devices and methods may be used to collect and process data from the plurality of measurement electrodes and may or may not generate an ECG waveform. For example, the signals from the physiological sensors 202 may facilitate the monitoring of certain cardiac characteristics (e.g., heart rate, arrhythmias, changes due to medications or surgery, function of pacemakers, heart size, etc.) and/or ECG waveform characteristics (e.g., timing of certain waves, intervals, complexes of the ECG waveform) by the DSP and/or user without generating a complete ECG waveform. In some examples, the controller may generate a subset of the ECG waveform (e.g., one or more of the P wave, QRS complex, PR interval, T wave, U wave). Moreover, examples of the disclosure include analysis and processing devices and methods configured for other types of physiological signal measurements including, but not limited to, EEG and EMG measurements or optical determination of parameters on blood constituents.

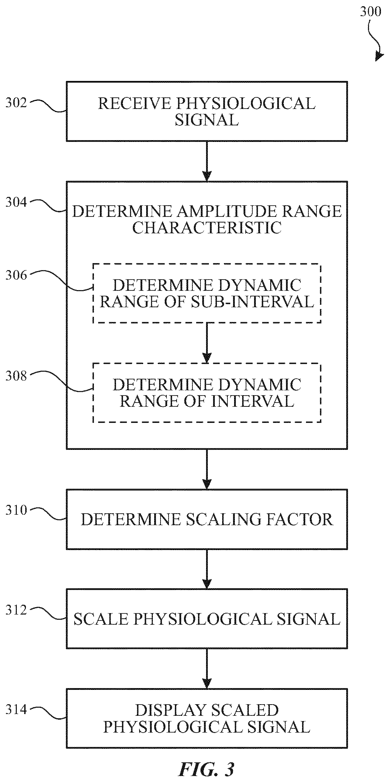

FIG. 3 illustrates an exemplary process 300 of processing a physiological signal (e.g., an ECG waveform) according to examples of the disclosure. At 302, one or more physiological signals can be received (e.g., by DSP 206) from the one or more physiological sensors (e.g., physiological sensor(s) 140, 160, 202). The one or more physiological sensors can be sampled by measuring electrical signals (e.g., ECG signals) from a person contacting the ECG sensor(s) electrodes to generate a physiological signal (e.g., ECG signal). The sampled ECG signal can be stored in a data buffer (e.g., data buffer 204) and one or more samples of the ECG signal in an interval (e.g., 0.5 second interval, 1 second interval, 3 second interval, 5 second interval, 10 second interval, 30 second interval, 60 second interval) can be accessed for processing at 302, 304, 306, 308, and 310 (e.g., by DSP 206).

At 304, an amplitude range characteristic (e.g., dynamic range) of the physiological signal (e.g., ECG signal) can be determined. In some examples, determining the amplitude range characteristic of a physiological signal can include determining the maximum amplitude value (e.g., voltage level) of the physiological signal in an interval, determining the minimum amplitude value (e.g., voltage level) of the physiological signal in an interval and determining the difference between the maximum and minimum amplitude value in the interval. In some examples, the maximum amplitude value and minimum amplitude value can correspond to the peak and trough of one beat in the physiological signal in the interval and thus the dynamic range can be the "peak-to-peak" amplitude of the beat in the physiological signal. In some examples, the maximum amplitude level and minimum amplitude value can correspond to the peak and trough of different beats in the physiological signal in the interval and thus the dynamic range can be determined across multiple beats in the physiological signal. For example, the peak of a first beat can be larger than the peak of a second beat and the trough of the first beat can be larger than the trough of the second beat. The dynamic range for the two beats can be determined as the difference between the peak of the first beat and the trough of the second beat).

In some examples, determining the amplitude range characteristic (304) optionally includes determining (306) the dynamic range of the physiological signal at multiple sub-intervals of time of an interval of time and determining (308) the dynamic range of the physiological signal for the interval of time based on the multiple sub-intervals. At 306, a dynamic range of the physiological signal for each of multiple sub-intervals of time can be determined (e.g., by DSP 206). In some examples, each sub-interval of time can be 0.25 seconds, 0.5 seconds, 1 second, or any other suitable time period less than the time period of the interval. In some examples, each sub-interval can be of the same duration. In some examples, the sub-intervals can be of different durations. In some examples, the sub-interval of time can span one or more beats in the physiological signal (e.g., representative of heart beats). In some examples, DSP 206 can analyze a 0.5 second sub-interval of the received ECG signal (e.g., including one beat) and determine the maximum signal amplitude value (e.g., voltage level) during the 0.5 second sub-interval (e.g., the peak of the beat), determine the minimum signal amplitude value (e.g., voltage level) during the 0.5 second sub-interval (e.g., the trough of the beat), and calculate the dynamic range as the difference between the maximum signal amplitude value and the minimum signal amplitude value for the 0.5 second sub-interval. This determination of the dynamic range can be repeated for one or more additional 0.5 second sub-intervals in the interval (e.g., two 0.5 second sub-intervals in a 1 second interval, four 0.5 second sub-intervals in a 2 second interval, etc.).

At 308, the dynamic range of the physiological signal for the interval of time can optionally be determined (e.g., by DSP 206) based on the dynamic range determined at the multiple sub-intervals of time (e.g., at 306). In some examples, the dynamic range of the physiological signal can be determined using a plurality of dynamic ranges determined at sub-intervals of time (e.g., 6 consecutive 0.5 second sub-intervals for a total of 3 seconds). The dynamic ranges for each of the sub-intervals can be aggregated to represent the dynamic range of the entire interval of time. In some examples, aggregating the dynamic range of each of the sub-intervals of time can be performed by taking an average of the dynamic range of each of the sub-intervals in the interval. In some examples, the average can be an arithmetic mean (weighted or unweighted), mode, or median value of the dynamic range of each of the sub-intervals. For example, a median of the dynamic ranges of the 6 consecutive 0.5 second sub-intervals can be determined to represent the dynamic range of the 3 second interval encompassing the 6 sub-intervals. Other methods of aggregating the dynamic range of the sub-intervals can be used, such as calculating a probability mass function, a cumulative distribution function, or any other suitable statistical analysis method.

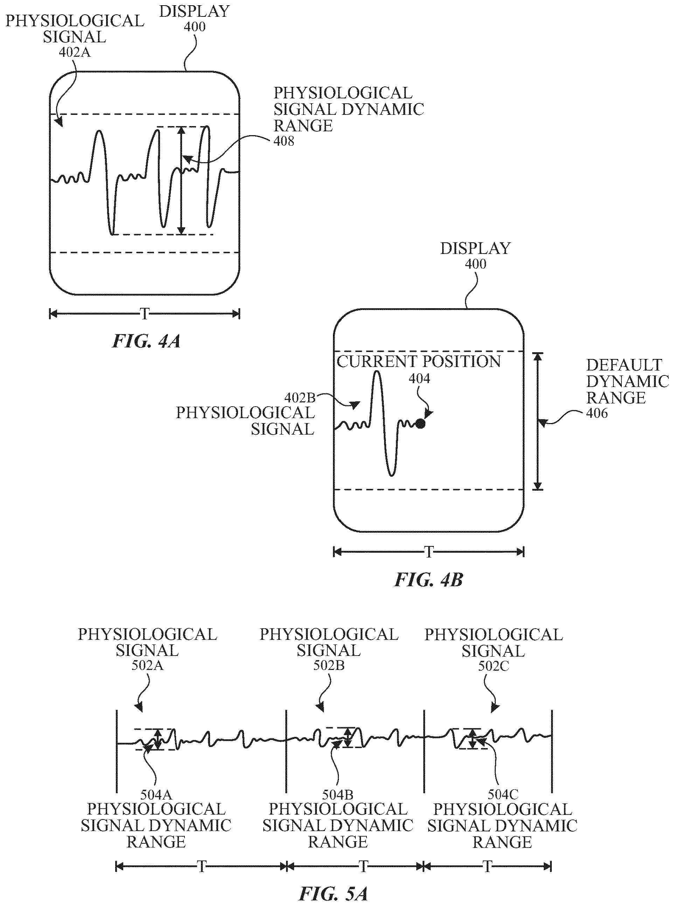

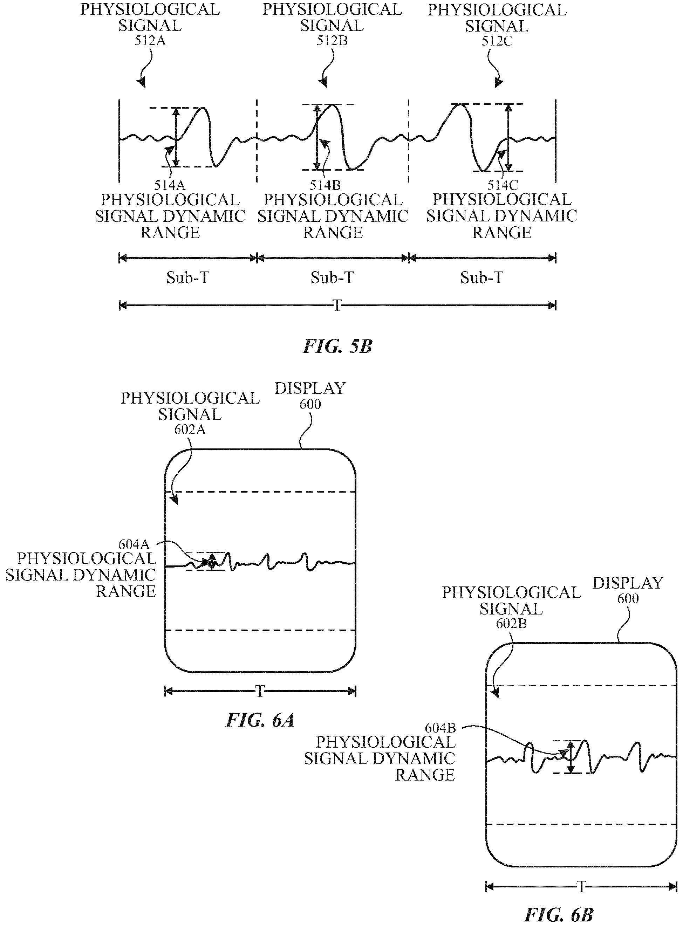

At 310, a scaling factor for the physiological signal can be determined (e.g., by DSP 206). In some examples, based on the physiology of the user (e.g., size or shape of the heart), the physiological signal received by the one or more physiological sensors can be of a typical amplitude, smaller amplitude or of larger amplitude. In some examples, the placement of the one or more physiological sensors can affect the size and shape of the received physiological signal. A scaling factor can be determined for these small and/or large amplitude signals to translate the raw physiological signal amplitude (or dynamic range) into a signal with amplitude (or dynamic range) more suitable for display. In some examples, the scaling can be performed in real-time. In such examples, the dynamic range of the physiological signal during a particular interval of time can be analyzed to determine whether to perform scaling of the physiological signal, and if so, can determine the appropriate scaling factor for the display of the physiological signal of that time interval. In some examples, the dynamic range of the physiological signal during a particular interval of time can be analyzed to determine whether to perform scaling of the physiological signal, and, if so, can determine the appropriate scaling factor for a subsequent time interval, as will be described in further detail with respect to FIGs. 5-8C.

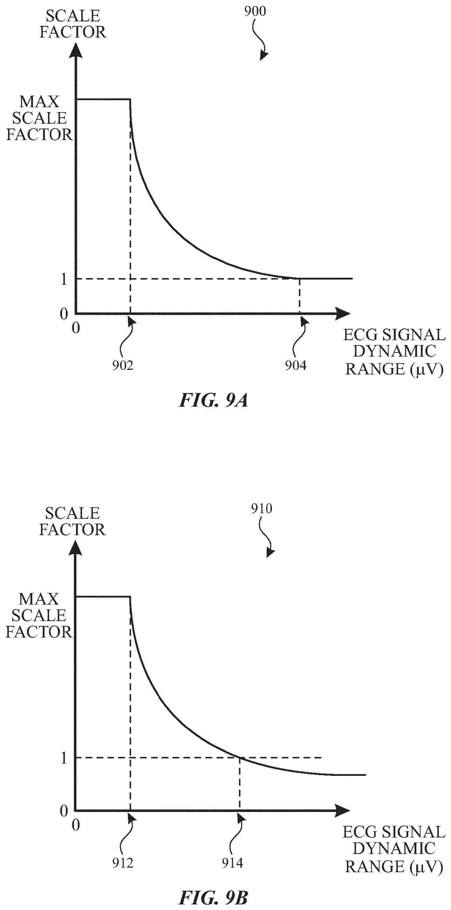

In some examples, the amplitude or dynamic range of the signal can be within a typical range and no scaling may be necessary (i.e., scaling factor of 1). In some examples, dynamic ranges above a threshold level and/or below a threshold level can be scaled to increase and/or decrease the size of the physiological signal, as will be described in more detail below with respect to FIGs. 9A-9B. At 312, the physiological signal can be scaled using the scaling factor determined at 310 (e.g., by DSP 206). In some examples, scaling the physiological signal can include multiplying the physiological signal by the scaling factor (e.g., amplifying the signal). In some examples, scaling factors (one or more different scaling factors) can be determined for each interval of time (e.g., 1 second, 3 seconds, 5 seconds) and applied to the physiological signal for display for a corresponding interval of time (e.g., in real-time to the current interval of time or to a subsequent interval of time). In some examples, one determined scaling factor can be applied to physiological signals for display for multiple intervals of time. For example, a scaling factor for a physiological signal session (e.g., 10 seconds, 30 seconds, 60 seconds, etc.) including multiple intervals of time can be determined during a first interval and can then be applied to the physiological signal for display for each interval in the session.

In some examples, determining a scaling factor at 310 and scaling the physiological signal at 312 can be skipped. For example, when the amplitude or dynamic range of the signal is within a predetermined range (e.g., corresponding to a scaling factor of 1 or no scaling), the determination and scaling of the physiological signal may be skipped. For example, in accordance with a determination that the amplitude range characteristic of the first physiological signal meets one or more criteria (such that scaling may be triggered) a scaling factor can be determined in accordance with the amplitude range characteristic of a physiological signal (at 310), a physiological signal (the same or a subsequent physiological signal) can be scaled based on the determined scaling factor (at 312), and the scaled physiological signal can be displayed on a display (at 314). In accordance with a determination that the amplitude range characteristic of the first physiological signal meets one or more criteria (such that scaling may not be triggered), the physiological signal can be displayed on the display (at 314) without determining a scaling factor (at 310) or scaling the physiological signal (at 312).

At 314, the scaled physiological signal can be displayed (e.g., on a display of the mobile device 136 or wearable device 150 or another device or display). In some examples, the device can display, on the display, the scaled or un-scaled physiological signal corresponding to a threshold time period. In some examples, the threshold time period can be equal to one interval of time of the physiological signal (e.g., 1 second, 3 seconds, 5 seconds) used to determine amplitude range characteristic and/or scaling factor at 304 and 310, respectively. In some examples, the threshold time period can be greater (e.g., a multiple of) or less than (e.g., a fraction of) the interval of time used to determine dynamic range and/or scaling factor. In some examples, the physiological signal for an interval of time can be displayed by sweeping. For example, the physiological signal can be traced from left to right, where the rate of tracing and sweeping can correspond to the timing of the waveform. In some examples, the sweep can be continuous and the physiological signal can continue to be traced such that the waveform appears to move in time (e.g., proportionately or substantially at the same rate at which the signal is generated by the user and received by the electrodes). In some examples, after tracing the physiological signal for the threshold time period, the display can refresh and being sweeping again. For example, at the end of the threshold time period (e.g., after the physiological signal reaches the end of the display), the display can be cleared and can begin sweeping by tracing the physiological signal for the next threshold time period.

FIGs. 4A-4B illustrate exemplary display of a physiological signal 402A-B on display 400 according to examples of the disclosure. In FIG. 4A, one interval of time of duration T (e.g., 1 second, 3 seconds, 5 seconds) of the physiological signal 402A (e.g., ECG signal) can be displayed on display 400. For example, the leftmost portion of physiological signal 402A can correspond to the start of the interval of time and the rightmost portion of the physiological signal 402A can correspond to the end of the interval of time. The displayed physiological signal 402A can represent the signal amplitude of the physiological signal (e.g., voltage level) with a dynamic range 408. As illustrated, dynamic range 408 of physiological signal 402A can correspond to the difference between the maximum amplitude of the peak of the third beat of physiological signal 402A and the minimum amplitude of the trough the first beat of physiological signal 402A. Default dynamic range 406, illustrated by the dotted lines on the display, is included for illustrative purposes only and can represent a range of values that can be displayed in the allotted space in the user interface on display 400. The default dynamic range 406 can contain the dynamic range of the physiological signal of most users without scaling described herein. As illustrated in FIG. 4A, physiological signal 402A can have dynamic range 408 within the default dynamic range 406. Default dynamic range 406 can be determined empirically such that the physiological signal of a threshold percentage (e.g., 80%, 95%) of users of the device have a dynamic range within a threshold value (e.g., within 50mV) of the default dynamic range. In some examples, the default dynamic range can correspond to a predetermined amount of the display height (e.g., the center 75% of the display height). In some examples, default dynamic range 406 can be between 0-1000 µV. In some examples, default dynamic range 406 can be different (e.g., -500 µV -1500 µV, - 1000 µV - 2000 µV, -1500 µV - 2500 µV). As primarily described herein, the default dynamic range can remain unchanged, and the scaling of physiological signals described herein can be achieved by multiplying the physiological signal by a scaling factor (e.g., increasing the amplitude of the signal, but leaving the display scale unchanged). However, it should be understood that scaling of the physiological signal on the display can alternatively be achieved by leaving the physiological signal unchanged while changing the scale on the display from the default dynamic range.

FIG. 4B illustrates an example tracing of physiological signal 402B for a time interval following the display of physiological signal 402A on display 400. After displaying a first interval of time of the physiological signal (e.g., as illustrated in Fig. 4A by physiological signal 402A), the display can refresh (e.g., clear the display), and begin tracing a second interval of time of the physiological signal, shown as physiological signal 402B, from left to right. Thus, the display can display a real-time or substantially real-time signal amplitude of the physiological signal. In some examples, the display of a physiological signal can be slightly delayed due to processing delays (e.g., 100 ms, 300 ms, 500 ms, etc.). In FIG. 4B, physiological signal 402B can be display by sweeping from the left to the right of the display. In some examples, a current position indicator 404 illustrated by an enlarged or otherwise emphasized indicator on physiological signal 402B can be displayed represent the current position (e.g., live position) of the sweep for the interval of time. In some examples, the display of physiological signal 402B can continue by sweeping to the right until reaching the end of the display at the end of the interval of time (e.g., 1 second, 3 seconds, 5 seconds). Although illustrated and described as spanning from the left end of display 400 to the right end of display 400, it should be understood that margins can be added between the physical ends of the display and the ends of physiological signal 402B displayed on display 400. In some examples, the physiological signal can be displayed in a display window or other graphical user interface occupying less than the full display area.

As described herein, in some examples, a physiological signal may be scaled to increase the displayed amplitude of the physiological signal. FIGs. 5-6C illustrate exemplary raw physiological signal measurements and corresponding display of the physiological signal measurements with or without scaling according to examples of the disclosure. FIG. 5A illustrates an example raw physiological signal measurement according to examples of the disclosure. FIG. 5A illustrates three consecutive time periods (intervals) of an example raw physiological signal (i.e., 502A-C), each with a duration T. Duration T can be equal to the interval of time of the physiological signal (e.g., 1 second, 3 seconds, 5 seconds) used to determine amplitude range characteristic and/or scaling factor (e.g., at 304 and 310 in FIG. 3, respectively). As shown in FIG. 5A, physiological signals 502A-C can have dynamic ranges 504A-C, respectively, which can be smaller than the default dynamic range of the display device. Thus, displaying raw physiological signals 502A-C on a display (such as on display 400) without scaling would display a small signal that may be difficult to interpret or lead to misinterpretation of the physiological signal (e.g., as compared with physiological signal 402A-B).

As described above, in some examples the processing of the physiological signal may include determining dynamic range of sub-intervals. FIG. 5B illustrates an example raw physiological signal measurement according to examples of the disclosure. FIG. 5B illustrates three consecutive sub-intervals (i.e. 512A-C) of an interval of an example raw physiological signal, each with a duration sub-T. For example, the three consecutive sub-intervals (e.g., 0.25 seconds, 0.5 seconds, 1 second) of physiological signal 512A, 512B, and 512C can constitute a physiological signal of one interval with duration T (e.g., 3 sub-intervals of 1 second for a total of a 3 second interval). For example, the example raw physiological signal illustrated in FIG. 5B can correspond to physiological signal 502A (or can correspond to another interval such as physiological signals 502B or 502C) in FIG. 5A. In some examples, physiological signal 512A corresponding to the first sub-interval can have a dynamic range 514A, physiological signal 512B corresponding to the second sub-interval can have a dynamic range 514B, and physiological signal 512C corresponding to the third sub-interval can have a dynamic range 514C. In some examples, the dynamic range of each sub-interval can be determined (e.g., at 306). In some examples, the dynamic ranges of each sub-interval can be aggregated to determine the dynamic range for the interval of time that comprises the three sub-intervals (e.g., at 308). For example, dynamic ranges 514A, 514B, and 514C for the respective sub-intervals of an interval of the physiological signal can be averaged to determine the dynamic range for the interval.

FIGs. 6A-6C illustrate an example display of physiological signals 502A-C, respectively. FIG. 6A illustrates an example display of physiological signal 502A on display 600. In FIG. 6A, the displayed physiological signal 602A is not scaled (e.g., corresponding to a scaling factor of 1 or no scaling) and displays a physiological signal with a dynamic range 604A, which can be equivalent to the dynamic range 504A of the raw signal 502A and smaller than the default dynamic range illustrated by the dotted lines. In some examples, physiological signal 502A in FIG. 5 (and displayed in FIG. 6A) can correspond to the first sampled time interval in a session of measuring the physiological signal before a scaling factor can be determined by processing the physiological signal. For this reason, in some examples, the first time interval in the session (e.g., such as 504A) can be displayed without scaling. In some examples, the first time interval can be scaled using a predetermined initial scaling factor. The predetermined initial scaling factor can be based on historical scaling factors. For example, a scaling factor or a mean, mode or median of scaling factors from a recent history of scaling factors (e.g., from one or more prior sessions) can be used. In some examples, an initial scaling factor for a user can be determined when the device (e.g., wearable device 150) is placed in contact with the user's skin and can be used for each session until the contact between the device and the user's skin is detected. In some examples, the initial scaling factor for the device can be determined when the device is initialized (e.g., during user set up of the device).

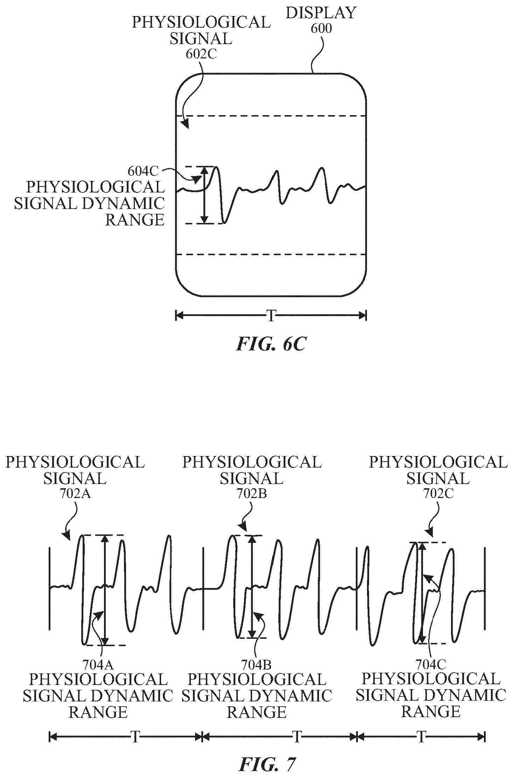

FIG. 6B illustrates an example display of raw physiological signal 502B on display 600. In FIG. 6B, physiological signal 602B can be scaled based on the dynamic range analysis (e.g., according to process 300) of physiological signal 502A, corresponding to the physiological signal during the first time period, resulting in a determined scaling factor for use for physiological signal 502B, corresponding to the physiological signal during the second time interval (i.e., scaling physiological signal 502B based on analysis of physiological signal 502A, the previous time period). As shown in FIG. 6B, the displayed scaled physiological signal 602B can have a dynamic range 604B larger than dynamic range 504B of raw physiological signal 502B, but the dynamic range 604B may still be smaller than the default dynamic range illustrated by dotted lines. FIG. 6C illustrates an example display of raw physiological signal 502C on display 600. In FIG. 6C, physiological signal 602C can be scaled based on the dynamic range analysis (e.g., according to process 300) of physiological signal 502A and/or physiological signal 502B, corresponding to the physiological signal during the first and second time periods, respectively, which can result in a different scaling factor for the third time interval than the scaling factor applied to the second time interval of the physiological signal. As shown in FIG. 6C, the physiological signal 502C can be scaled based on the scaling factor and results in a displayed physiological signal 602C with a dynamic range 604C that can be different (e.g., larger) than the dynamic range 604B of scaled physiological signal 602B displayed during the second time interval.

In some examples, physiological signal 602C can be scaled based on the dynamic range analysis (e.g., according to process 300) of physiological signal 502A (i.e., scaling physiological signal 502C based on analysis of physiological signal 502A, the signal from two time periods ago), without dynamic range analysis of physiological signal 502B. For example, a scaling factor can be determined at the start of a session and used for subsequent intervals of the physiological signals in the measurement. In some examples, physiological signal 602C can be scaled based on the dynamic range analysis of physiological signal 502B (i.e., scaling physiological signal 502C based on analysis of physiological signal 502B, the previous time period). For example, a scaling factor for an interval can be determined based on analysis of the dynamic range of the previous period. In some examples, physiological signal 602C can be scaled based on the dynamic range analysis of physiological signal 502A and 502B (i.e., scaling physiological signal 502C based on analysis of the previous two time periods). Scaling based on multiple time periods (e.g., the previous two time periods) can comprise determining the dynamic range value for each time period (e.g., according to 304 of process 300), and determining the average of the dynamic range values (e.g., mean, medium, mode, or other suitable aggregation method, such as a leaky aggregator). Such examples, can provide a level of hysteresis so that the scaling does not change drastically from interval to interval on the display. Although two intervals are described above, a history of scaling factors from multiple intervals can be used (e.g., three, ten, etc.) from one or more sessions. In some examples, the scaling factor can be graduated such that the displayed dynamic range of the physiological signals gradually increases (or decreased) from one time period to the next time period to reach an equilibrium displayed dynamic range. In other words, in some examples, to avoid abrupt changes in the displayed physiological signal, the scaling factor actually used to scale a physiological signal can be a percentage of the determined scaling factor and the percentage increases over one or more time periods to reach 100% of the determined scaling factor.

In some examples, scaling based on multiple time periods (intervals) can comprise determining the dynamic range value for each of the time periods according to process 300. For example, the dynamic range value for each of the multiple time periods can be determined for each interval or dynamic range values can be determined for each sub-interval of time and aggregated (e.g., at 306 and 308) for each interval. In some examples, scaling based on multiple time periods can comprise determining the scaling factor for each time period (e.g., according to process 300) and determining the average of the scaling factors for the multiple time periods (e.g., mean, medium, mode, or other suitable aggregation method, such as a leaky aggregator). In some examples, the scaling factor determined from analyzing one previous time period can be equal to the scaling factor determined from analyzing multiple previous time periods. In some examples, the scaling factor determined by analyzing different time periods can be different.

In some examples, the scaling factor can be selected to normalize the dynamic range and display the physiological signal on the display with the default dynamic range. However, normalizing the physiological signal may give a misimpression about the amplitude/dynamic range of the physiological signal. For example, a user with a small physiological signal may not be aware of this condition. In some examples, the scaled physiological signal may not be normalized to the default dynamic range and the scaled physiological signal can be smaller than the default dynamic range (as shown in FIG. 6C). In such an example, displaying a physiological signal smaller than the default dynamic range can indicate to the user of the device that the raw physiological signal can be small (e.g., either due to physiology of the user or environmental factors such as respiration, movement, or stability of contact between the physiological sensor and the user) relative to most users. Likewise, in some examples, displaying a physiological signal larger than the default dynamic range can indicate to the user of the device that the raw physiological signal can be large relative to most users.

FIG. 7 illustrates an example raw physiological signal according to examples of the disclosure. FIG. 7 illustrates three consecutive time periods of an example raw physiological signal (i.e., 702A-C), each with a duration T. Duration T can be equal to the interval of time of the physiological signal (e.g., 1 second, 3 seconds, 5 seconds) used to determine amplitude range characteristic and/or scaling factor (e.g., at 304 and 310 in FIG. 3, respectively). As shown in FIG. 7, physiological signals 702A-C can have dynamic ranges 704A-C, respectively, which can be larger than the default dynamic range of the display device. Thus, displaying raw physiological signals 702A-C on a display (such as on display 400) without scaling would display a large signal that potentially extends beyond the display range of the display (or the graphical user interface for displaying the physiological signal). In some examples, such a large signal could cause the signal to be clipped.

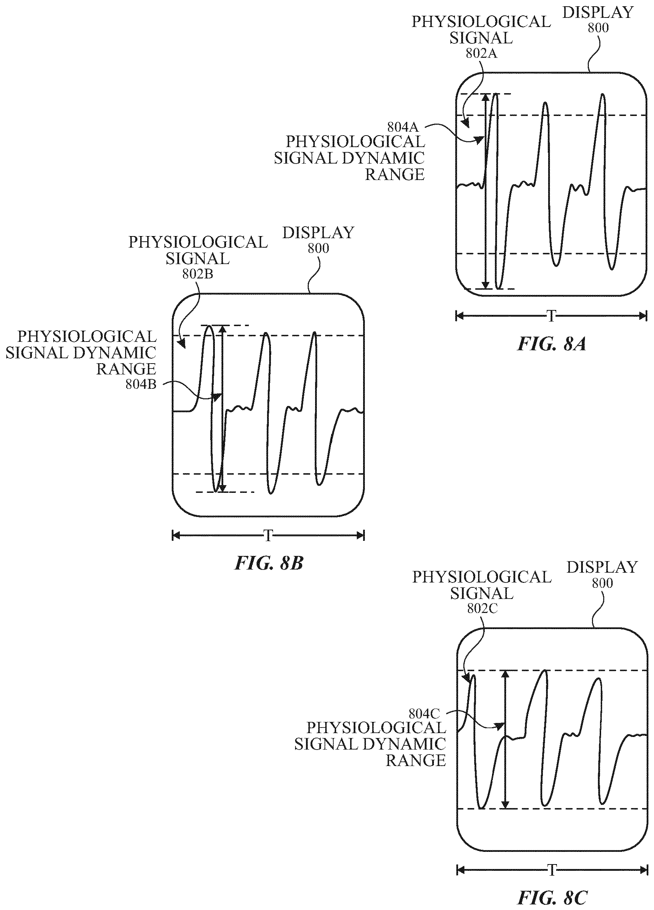

FIGs. 8A-8C illustrate an example display of physiological signals 702A-C, respectively. FIG. 8A illustrates an example display of physiological signal 702A on display 800. In FIG. 8A, the displayed physiological signal 802A is not scaled (e.g., corresponding to a scaling factor of 1 or no scaling) and displays a physiological signal with a dynamic range 804A, which can be equivalent to the dynamic range 704A of the raw signal 702A and larger than the dynamic range illustrated by the dotted lines. In some examples, physiological signal 802A in FIG. 7 (and displayed in FIG. 8A) can correspond to the first sampled time interval in a session of measuring the physiological signal before a scaling factor can be determined by processing the physiological signal. For this reason, in some examples, the first time interval in the session (e.g., such as 704A) can be displayed without scaling. In some examples, the first time interval can be scaled using a predetermined scaling factor (e.g., determined prior to measuring the physiological signal to be scaled) based on historical scaling factors.

FIG. 8B illustrates an example display of raw physiological signal 702B on display 800. In FIG. 8B, physiological signal 802B can be scaled based on the dynamic range analysis (e.g., according to process 300) of physiological signal 702A, corresponding to the physiological signal during the first time period, resulting in a determined scaling factor for use for physiological signal 702B, corresponding to the physiological signal during the second time interval (i.e., scaling physiological signal 702B based on analysis of physiological signal 702A, the previous time period). As shown in FIG. 8B, the displayed scaled physiological signal 802B can have a dynamic range 804B smaller than dynamic range 704B of raw physiological signal 702B, but the dynamic range 804B may still be larger than the default dynamic range illustrated by dotted lines. FIG. 8C illustrates an example display of raw physiological signal 702C on display 800. In FIG. 8C, physiological signal 802C can be scaled based on the dynamic range analysis (e.g., according to process 300) of physiological signal 702A and/or physiological signal 702B, corresponding to the physiological signal during the first and second time periods, respectively, which can result in a different scaling factor for the third time interval than the scaling factor applied to the second time interval of the physiological signal. As shown in FIG. 8C, the physiological signal can scaled based on the scaling factor and results in a displayed physiological signal 802C with a dynamic range 804C that can be smaller than the dynamic range 804B of scaled physiological signal 802B displayed during the second time interval and can be normalized to the default dynamic range illustrated by the dotted lines. In some examples, the scaled physiological signal can be not normalized to the default range and be displayed larger than the default dynamic range. In such an example, displaying a physiological signal larger than the default dynamic range can indicate to the user of the device that the raw physiological signal is large compared with the general population. In some examples, the scaling factor applied to the physiological signal can be graduated, similar to that described above with respect to FIGs. 6A-6C. In some examples, the scaling factor can be determined based on analysis of one or more time periods (current or previous), similar to that described above with respect to FIGs. 6A-6C.

As described herein, in some examples, an entire interval of the physiological signal being displayed on the display can be scaled by one scaling factor that can be determined based on analysis of a previous time interval. In some examples, the physiological signal being displayed can be scaled using one or more scaling factors. In some examples, the physiological signal being displayed can be divided into a plurality of segments (i.e., 2 segments, 3 segments, 6 segments, etc.), and a newly calculated scaling factor can be used for each of the segments. In some examples, the plurality of segments can be the same length of time or different lengths of time. For example, a 3-second physiological signal being displayed can be divided into two equal halves (i.e., each with a 1.5-second duration). The scaling factor applied to the first half of the 3-second physiological signal being displayed can be determined based on analysis of a 3-second interval of the raw physiological signal immediately preceding the first half of the 3-second physiological signal being displayed. The scaling factor applied to the second half of the 3-second physiological signal being displayed can be determined based on analysis of a 3-second interval of the raw physiological signal immediately preceding the second half of the 3 second physiological signal being displayed. Thus, the scaling factors can be determined for the 3-second physiological signal being displayed based on a sliding window, where 1.5 seconds of the raw physiological signal processed to determine the two scaling factors can overlap. Although in the above example, the physiological signal for an interval was displayed using two scaling factors, more scaling factors can be used in some examples. In some examples, new scaling factors can be calculated for an interval and applied continuously or in a piecewise manner to the physiological signal being displayed. In some examples, the physiological signal can be divided such that the segments have the same length of time as the sub-intervals of time for which the dynamic ranges can be determined at 306, as shown in FIG. 5B. As described above, in some examples, the scaling factors actually applied can be graduated to avoid abrupt visual discontinuities or changes in the displayed physiological signal.

FIGs. 9A-9C illustrate example scaling factor functions according to examples of the disclosure. In some examples, at a certain predetermined range of values for the dynamic range of the physiological signal, a predetermined maximum scale factor can be applied. For example, using a maximum scaling factor can preserve information regarding a small physiological signal by avoiding normalizing the dynamic range of the physiological signal, which may be desirable to illustrate that a user's physiological signal is relatively small (e.g., less than a threshold percentile of the general population, such as 5%, 3%, 1%, etc.). For example, as illustrated in FIGs. 9A-9C, a maximum scaling factor can be applied to scale the respective physiological signal for physiological signals with a dynamic range below a threshold dynamic range indicated by minimum dynamic range value 902, 912 and 922. In some examples, at dynamic ranges above minimum dynamic range value 902, 912 and 922, the scaling factor applied to the physiological signal can vary as a function of the dynamic range. For example, as illustrated in FIGs. 9A-9C, the scaling factor may decrease exponentially from the maximum scaling factor as the dynamic range of the physiological range increases. In some examples, the scaling factor function can decrease linearly as the dynamic range of the physiological range increases. Although shown as a continuous function, it should be understood that the scaling factors can be applied in a step-wise manner, such that the scaling factors are integer multipliers. In some examples, the step-wise function can use a floor or step to reach the desired scaling factor. Other linear or nonlinear functions can be used without departing from the scope of the disclosure. In some examples, as illustrated in FIGs. 9A-9B, scaling factor function 900 and 910 asymptotically approaches a minimum scaling factor. In some examples, as illustrated in FIG. 9A, the minimum scaling factor can be 1, which can indicate that no scaling is applied at dynamic ranges above a dynamic range threshold 904. In some examples, as illustrated in FIG. 9B, the minimum scaling factor can be less than 1 at dynamic ranges above a dynamic range value 914, which can indicate that scaling can decrease the dynamic range or amplitude of the physiological signal for display. For example, scaling factor function 910 can asymptotically approach a minimum scaling factor value less than 1 but above 0 (i.e. a fraction or decimal value). The physiological signal can be scaled upward for dynamic ranges less than dynamic range value 914, and the physiological signal can be scaled downward for dynamic ranges greater than dynamic range value 914. In some examples, the minimum scaling factor can preserve information regarding clipping of a large physiological signal by avoiding normalizing the dynamic range of the physiological signal, which may be desirable to illustrate that a user's physiological signal is relatively large (e.g., greater than a threshold percentile of the general population, such as 95%, 99%, etc.).

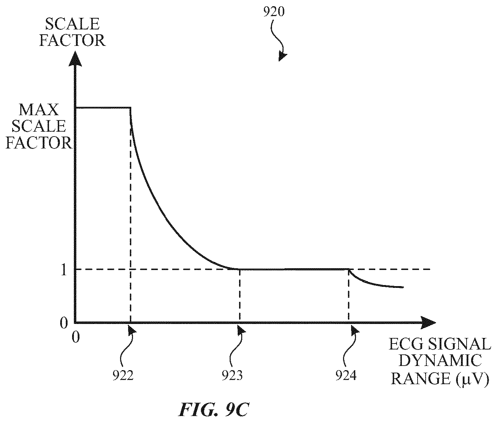

In some examples, as illustrated in FIG. 9C, the scaling factor function 920 asymptotically approached an intermediate scaling factor. In some examples, as illustrate in FIG. 9C, the intermediate scaling factor can be 1, which can indicate that no scaling is applied at dynamic ranges between dynamic range thresholds 923 and 924. In some examples, as illustrated in FIG. 9C, the minimum scaling factor can be less than 1 at dynamic ranges above a dynamic range value 924, which can indicate that scaling can decrease the dynamic range or amplitude of the physiological signal for display. For example, scaling factor function 920 can asymptotically approach a minimum scaling factor value less than 1 but above 0 (i.e. a fraction or decimal value). In some examples, dynamic range thresholds 923 and 924 can be set such that most physiological signals experience no scaling (e.g., a threshold percentage (e.g., 80%, 95%) of users of the device have a dynamic range without any scaling). This may be desirable to show a consistent scale without scaling of the physiological signal) over time under most operating conditions. Scaling of the physiological signal can be reserved for edge cases where an especially small or large physiological signal may impact the ability to view the physiological using the default scale (e.g., because the signal may be too small or may be clipped).

As discussed above, aspects in of the present technology include the gathering and use of physiological information. The technology may be implemented along with technologies that involve gathering personal data that relates to the user's health and/or uniquely identifies or can be used to contact or locate a specific person. Such personal data can include demographic data, date of birth, location-based data, telephone numbers, email addresses, home addresses, and data or records relating to a user's health or level of fitness (e.g., vital signs measurements, medication information, exercise information, etc.).

The present disclosure recognizes that a user's personal data, including physiological information, such as data generated and used by the present technology, can be used to the benefit of users. For example, a user's heart rate may allow a user to track or otherwise gain insights about their health or fitness levels.

The present disclosure contemplates that the entities responsible for the collection, analysis, disclosure, transfer, storage, or other use of such personal data will comply with well-established privacy policies and/or privacy practices. In particular, such entities should implement and consistently use privacy policies and practices that are generally recognized as meeting or exceeding industry or governmental requirements for maintaining personal information data private and secure. Such policies should be easily accessible by users, and should be updated as the collection and/or use of data changes. Personal information from users should be collected for legitimate and reasonable uses of the entity and not shared or sold outside of those legitimate uses. Further, such collection/sharing should require receipt of the informed consent of the users. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. The policies and practices may be adapted depending on the geographic region and/or the particular type and nature of personal data being collected and used.

Despite the foregoing, the present disclosure also contemplates embodiments in which users selectively block the collection of, use of, or access to, personal data, including physiological information. For example, a user may be able to disable hardware and/or software elements that collect physiological information. Further, the present disclosure contemplates that hardware and/or software elements can be provided to prevent or block access to personal data that has already been collected. Specifically, users can select to remove, disable, or restrict access to certain health-related applications collecting users' personal health or fitness data.

Therefore, according to the above, some examples of the disclosure are directed to a method. The method can comprise measuring a first physiological signal using a physiological sensor during a first time interval; measuring a second physiological signal using the physiological sensor during a second time interval; determining an amplitude range characteristic of the first physiological signal; in accordance with a determination that the amplitude range characteristic of the first physiological signal meets one or more criteria: determining a first scaling factor in accordance with the amplitude range characteristic of the first physiological signal; scaling the second physiological signal based on the determined first scaling factor; and displaying the scaled second physiological signal on a display; and in accordance with a determination that the amplitude range characteristic of the first physiological signal fails to meet the one or more criteria: displaying the second physiological signal on the display.

Additionally or alternatively, in some examples, determining the amplitude range characteristic of the first physiological signal can comprise determining a dynamic range of the first physiological signal. Additionally or alternatively, in some examples, determining the dynamic range of the first physiological signal can comprise determining a difference between a maximum amplitude value of the first physiological signal during the first time interval and a minimum amplitude value of the first physiological signal during the first time interval. Additionally or alternatively, in some examples, the first time interval can comprise a plurality of sub-intervals and determining the dynamic range of the first physiological signal can comprise: determining a dynamic range of each of the plurality of sub-intervals; and determining the dynamic range of the first physiological signal based on the dynamic range of each of the plurality of sub-intervals. Additionally or alternatively, in some examples, determining the dynamic range of the first physiological signal based on the dynamic range of each of the plurality of sub-intervals can comprise determining an arithmetic mean, a mode, or a median of the dynamic range of each of the plurality of sub-intervals. Additionally or alternatively, in some examples, the one or more criteria can comprise a criterion that requires that the amplitude range characteristic of the first physiological signal is below a threshold amplitude range characteristic.

Additionally or alternatively, in some examples, determining the first scaling factor in accordance with the amplitude range characteristic of the first physiological signal can comprise: in accordance with a determination that the amplitude range characteristic of the first physiological signal is below a second threshold amplitude range characteristic, determining the first scaling factor as a maximum scaling factor; and in accordance with a determination that the amplitude range characteristic of the first physiological signal is above the second threshold amplitude range characteristic and below the second amplitude threshold, determining a scaling factor between the maximum scaling factor and a minimum scaling factor. Additionally or alternatively, in some examples, the method can further comprise scaling the first physiological signal based on a predetermined scaling factor; and displaying the scaled first physiological signal on the display. Additionally or alternatively, in some examples, the predetermined scaling factor can comprise a scaling factor determined from one or more previous sessions or a scaling factor determined during initialization.

Additionally or alternatively, in some examples, the method can further comprise measuring a third physiological signal using the physiological sensor during a third time interval; determining an amplitude range characteristic of the second physiological signal; in accordance with a determination that the amplitude range characteristic of the second physiological signal meets the one or more first criteria: determining a second scaling factor, different from the first scaling factor, in accordance with at least the amplitude range characteristic of the second physiological signal; scaling the third physiological signal based on the determined second scaling factor; and displaying the scaled third physiological signal on the display. Additionally or alternatively, in some examples, determining the second scaling factor in accordance with at least the amplitude range characteristic of the second physiological signal can comprise determining the second scaling factor based on an arithmetic mean, median or mode of the amplitude range characteristic of the second physiological signal and the amplitude range characteristic of the first physiological signal. Additionally or alternatively, in some examples, the first interval and the second interval can be consecutive time periods.

Some examples of the disclosure are directed to an electronic device. The electronic device can comprise a physiological sensor and one or more processing circuits coupled to the physiological sensor. The one or more processing circuits can be configured to measure a first physiological signal using the physiological sensor during a first time interval; measure a second physiological signal using the physiological sensor during a second time interval; determine an amplitude range characteristic of the first physiological signal; in accordance with a determination that the amplitude range characteristic of the first physiological signal meets one or more criteria: determine a first scaling factor in accordance with the amplitude range characteristic of the first physiological signal; scale the second physiological signal based on the determined first scaling factor; and display the scaled second physiological signal on a display; and in accordance with a determination that the amplitude range characteristic of the first physiological signal fails to meet the one or more criteria: display the second physiological signal on the display.

Additionally or alternatively, in some examples, determining the amplitude range characteristic of the first physiological signal can comprise determining a dynamic range of the first physiological signal. Additionally or alternatively, in some examples, determining the dynamic range of the first physiological signal can comprise determining a difference between a maximum amplitude value of the first physiological signal during the first time interval and a minimum amplitude value of the first physiological signal during the first time interval. Additionally or alternatively, in some examples, the first time interval can comprise a plurality of sub-intervals and determining the dynamic range of the first physiological signal can comprise: determining a dynamic range of each of the plurality of sub-intervals; and determining the dynamic range of the first physiological signal based on the dynamic range of each of the plurality of sub-intervals. Additionally or alternatively, in some examples, determining the dynamic range of the first physiological signal based on the dynamic range of each of the plurality of sub-intervals can comprise determining an arithmetic mean, a mode, or a median of the dynamic range of each of the plurality of sub-intervals. Additionally or alternatively, in some examples, the one or more criteria can comprise a criterion that requires that the amplitude range characteristic of the first physiological signal is below a threshold amplitude range characteristic.

Additionally or alternatively, in some examples, determining the first scaling factor in accordance with the amplitude range characteristic of the first physiological signal can comprise: in accordance with a determination that the amplitude range characteristic of the first physiological signal is below a second threshold amplitude range characteristic, determining the first scaling factor as a maximum scaling factor; and in accordance with a determination that the amplitude range characteristic of the first physiological signal is above the second threshold amplitude range characteristic and below the second amplitude threshold, determining a scaling factor between the maximum scaling factor and a minimum scaling factor. Additionally or alternatively, in some examples, the one or more processing circuits can be further configured to scale the first physiological signal based on a predetermined scaling factor; and display the scaled first physiological signal on the display. Additionally or alternatively, in some examples, the predetermined scaling factor can comprise a scaling factor determined from one or more previous sessions or a scaling factor determined during initialization.

Additionally or alternatively, in some examples, the one or more processing circuits can be further configured to measure a third physiological signal using the physiological sensor during a third time interval; determine an amplitude range characteristic of the second physiological signal; in accordance with a determination that the amplitude range characteristic of the second physiological signal meets the one or more first criteria: determine a second scaling factor, different from the first scaling factor, in accordance with at least the amplitude range characteristic of the second physiological signal; scale the third physiological signal based on the determined second scaling factor; and display the scaled third physiological signal on a display. Additionally or alternatively, in some examples, determining the second scaling factor in accordance with at least the amplitude range characteristic of the second physiological signal can comprise determining the second scaling factor based on an arithmetic mean, median or mode of the amplitude range characteristic of the second physiological signal and the amplitude range characteristic of the first physiological signal. Additionally or alternatively, in some examples, the first interval and the second interval can be consecutive time periods.

Some examples of the disclosure are directed to a non-transitory computer readable storage medium. The non-transitory computer readable storage medium can store instructions, which when executed by a device comprising a physiological sensor and one or more processing circuits, cause the one or more processing circuits to perform a method. In some examples, the method can comprise measuring a first physiological signal using a physiological sensor during a first time interval; measuring a second physiological signal using the physiological sensor during a second time interval; determining an amplitude range characteristic of the first physiological signal; in accordance with a determination that the amplitude range characteristic of the first physiological signal meets one or more criteria: determining a first scaling factor in accordance with the amplitude range characteristic of the first physiological signal; scaling the second physiological signal based on the determined first scaling factor; and displaying the scaled second physiological signal on a display; and in accordance with a determination that the amplitude range characteristic of the first physiological signal fails to meet the one or more criteria: displaying the second physiological signal on the display.

Additionally or alternatively, in some examples, determining the amplitude range characteristic of the first physiological signal can comprise determining a dynamic range of the first physiological signal. Additionally or alternatively, in some examples, determining the dynamic range of the first physiological signal can comprise determining a difference between a maximum amplitude value of the first physiological signal during the first time interval and a minimum amplitude value of the first physiological signal during the first time interval. Additionally or alternatively, in some examples, the first time interval can comprise a plurality of sub-intervals and determining the dynamic range of the first physiological signal can comprise: determining a dynamic range of each of the plurality of sub-intervals; and determining the dynamic range of the first physiological signal based on the dynamic range of each of the plurality of sub-intervals. Additionally or alternatively, in some examples, determining the dynamic range of the first physiological signal based on the dynamic range of each of the plurality of sub-intervals can comprise determining an arithmetic mean, a mode, or a median of the dynamic range of each of the plurality of sub-intervals. Additionally or alternatively, in some examples, the one or more criteria can comprise a criterion that requires that the amplitude range characteristic of the first physiological signal is below a threshold amplitude range characteristic.

Additionally or alternatively, in some examples, determining the first scaling factor in accordance with the amplitude range characteristic of the first physiological signal can comprise: in accordance with a determination that the amplitude range characteristic of the first physiological signal is below a second threshold amplitude range characteristic, determining the first scaling factor as a maximum scaling factor; and in accordance with a determination that the amplitude range characteristic of the first physiological signal is above the second threshold amplitude range characteristic and below the second amplitude threshold, determining a scaling factor between the maximum scaling factor and a minimum scaling factor. Additionally or alternatively, in some examples, the method can further comprise scaling the first physiological signal based on a predetermined scaling factor; and displaying the scaled first physiological signal on the display. Additionally or alternatively, in some examples, the predetermined scaling factor can comprise a scaling factor determined from one or more previous sessions or a scaling factor determined during initialization.

Additionally or alternatively, in some examples, the method can further comprise measuring a third physiological signal using the physiological sensor during a third time interval; determining an amplitude range characteristic of the second physiological signal; in accordance with a determination that the amplitude range characteristic of the second physiological signal meets the one or more first criteria: determining a second scaling factor, different from the first scaling factor, in accordance with at least the amplitude range characteristic of the second physiological signal; scaling the third physiological signal based on the determined second scaling factor; and displaying the scaled third physiological signal on the display. Additionally or alternatively, in some examples, determining the second scaling factor in accordance with at least the amplitude range characteristic of the second physiological signal can comprise determining the second scaling factor based on an arithmetic mean, median or mode of the amplitude range characteristic of the second physiological signal and the amplitude range characteristic of the first physiological signal. Additionally or alternatively, in some examples, the first interval and the second interval can be consecutive time periods.

Although examples of this disclosure have been fully described with reference to the accompanying drawings, it is to be noted that various changes and modifications will become apparent to those skilled in the art. Such changes and modifications are to be understood as being included within the scope of examples of this disclosure as defined by the appended claims.

Although examples of this disclosure have been fully described with reference to the accompanying drawings, it is to be noted that various changes and modifications will become apparent to those skilled in the art.

## Claims

1. A method comprising:
measuring (302) a first physiological signal using a physiological sensor (140, 160, 202) during a first time interval;
measuring (302) a second physiological signal using the physiological sensor during a second time interval, wherein the first physiological signal and the second physiological signal correspond to respective first and second electrocardiogram signals;
determining (304) an amplitude range characteristic of the first physiological signal, wherein the amplitude range characteristic of the first physiological signal is a dynamic range;
in accordance with a determination that the amplitude range characteristic of the first physiological signal meets one or more criteria, the one or more criteria comprising a criterion that requires that the amplitude range characteristic of the first physiological signal is below a first threshold amplitude range characteristic (923):
determining (310) a first scaling factor in accordance with the amplitude range characteristic of the first physiological signal, wherein determining the first scaling factor in accordance with the amplitude range characteristic of the first physiological signal comprises:
in accordance with a determination that the amplitude range characteristic of the first physiological signal is below a second threshold amplitude range characteristic (922), determining the first scaling factor to be a predetermined maximum scaling factor; and
in accordance with a determination that the amplitude range characteristic of the first physiological signal is above the second threshold amplitude range characteristic (922) and below the first threshold amplitude range characteristic (923), determining the first scaling factor to be between the predetermined maximum scaling factor and a minimum scaling factor;
scaling (312) the second physiological signal based on the determined first scaling factor; and
displaying (314) the scaled second physiological signal on a display (400, 600, 800); and
in accordance with a determination that the amplitude range characteristic of the first physiological signal fails to meet the one or more criteria:
displaying the second physiological signal on the display.

2. The method of claim 1, wherein determining (304) the amplitude range characteristic of the first physiological signal comprises determining a difference between a maximum amplitude value of the first physiological signal during the first time interval and a minimum amplitude value of the first physiological signal during the first time interval.

3. The method of claim 1, wherein the first time interval comprises a plurality of sub-intervals and wherein determining (304) the amplitude range characteristic of the first physiological signal comprises:
determining a dynamic range of each of the plurality of sub-intervals; and
determining the dynamic range of the first physiological signal based on the dynamic range of each of the plurality of sub-intervals.

4. The method of claim 3, wherein determining (304) the amplitude range characteristic of the first physiological signal based on the dynamic range of each of the plurality of sub-intervals comprises determining an arithmetic mean, a mode, or a median of the dynamic range of each of the plurality of sub-intervals.

5. The method of any preceding claim, further comprising:
scaling the first physiological signal based on a predetermined scaling factor; and
displaying the scaled first physiological signal on the display.

6. The method of claim 5, wherein the predetermined scaling factor comprises a scaling factor determined from one or more previous sessions or a scaling factor determined during initialization.

7. The method of any preceding claim, further comprising:
measuring a third physiological signal using the physiological sensor during a third time interval;
determining an amplitude range characteristic of the second physiological signal;
in accordance with a determination that the amplitude range characteristic of the second physiological signal meets the one or more criteria:
determining a second scaling factor, different from the first scaling factor, in accordance with at least the amplitude range characteristic of the second physiological signal;
scaling the third physiological signal based on the determined second scaling factor; and
displaying the scaled third physiological signal on the display.

8. The method of claim 7, wherein:
determining the second scaling factor in accordance with at least the amplitude range characteristic of the second physiological signal comprises determining the second scaling factor based on an arithmetic mean, median or mode of the amplitude range characteristic of the second physiological signal and the amplitude range characteristic of the first physiological signal.

9. The method of any preceding claim, wherein the first interval and the second interval are consecutive time periods.

10. An electronic device comprising:
a physiological sensor (140, 160, 202); and
one or more processing circuits coupled to the physiological sensor, the one or more processing circuits configured to perform a method of any of claims 1-9.

11. A computer readable medium comprising instructions, which when executed by a device comprising a physiological sensor (140, 160, 202) and one or more processing circuits, cause the one or more processing circuits to perform a method of any of claims 1-9.

## Patentansprüche

1. Ein Verfahren, umfassend:
Messen (302) eines ersten physiologischen Signals unter Verwendung eines physiologischen Sensors (140, 160, 202) während eines ersten Zeitintervalls;
Messen (302) eines zweiten physiologischen Signals unter Verwendung des physiologischen Sensors während eines zweiten Zeitintervalls, wobei das erste physiologische Signal und das zweite physiologische Signal jeweiligen ersten und zweiten Elektrokardiogrammsignalen entsprechen;
Bestimmen (304) einer Amplitudenbereichscharakteristik des ersten physiologischen Signals, wobei die Amplitudenbereichscharakteristik des ersten physiologischen Signals ein dynamischer Bereich ist;
gemäß einer Bestimmung, dass die Amplitudenbereichscharakteristik des ersten physiologischen Signals ein oder mehrere Kriterien erfüllt,
wobei das eine oder die mehreren Kriterien ein Kriterium umfassen, das erfordert, dass die Amplitudenbereichscharakteristik des ersten physiologischen Signals unter einer ersten Schwellenwertamplitudenbereichscharakteristik (923) liegt:
Bestimmen (310) eines ersten Skalierungsfaktors gemäß der Amplitudenbereichscharakteristik des ersten physiologischen Signals, wobei das Bestimmen des ersten Skalierungsfaktors gemäß der Amplitudenbereichscharakteristik des ersten physiologischen Signals umfasst:
gemäß einer Bestimmung, dass die Amplitudenbereichscharakteristik des ersten physiologischen Signals unter einer zweiten Schwellenwertamplitudenbereichscharakteristik (922) liegt, Bestimmen, dass der erste Skalierungsfaktor ein vorbestimmter maximaler Skalierungsfaktor ist; und
gemäß einer Bestimmung, dass die Amplitudenbereichscharakteristik des ersten physiologischen Signals über der zweiten Schwellenwertamplitudenbereichscharakteristik (922) und unter der ersten Schwellenwertamplitudenbereichscharakteristik (923) liegt, Bestimmen, dass der erste Skalierungsfaktor zwischen dem vorbestimmten maximalen Skalierungsfaktor und einem minimalen Skalierungsfaktor liegt;
Skalieren (312) des zweiten physiologischen Signals basierend auf dem bestimmten ersten Anzeigen (314) des skalierten zweiten physiologischen Signals auf einer Anzeige (400, 600, 800); und
gemäß einer Bestimmung, dass die Amplitudenbereichscharakteristik des ersten physiologischen Signals das eine oder die mehreren Kriterien nicht erfüllt:
Anzeigen des zweiten physiologischen Signals auf der Anzeige.

2. Das Verfahren nach Anspruch 1, wobei Bestimmen (304) der Amplitudenbereichscharakteristik des ersten physiologischen Signals das Bestimmen einer Differenz zwischen einem maximalen Amplitudenwert des ersten physiologischen Signals während des ersten Zeitintervalls und einem minimalen Amplitudenwert des ersten physiologischen Signals während des ersten Zeitintervalls umfasst.

3. Das Verfahren nach Anspruch 1, wobei das erste Zeitintervall eine Vielzahl von Unterintervallen umfasst und wobei Bestimmen (304) der Amplitudenbereichscharakteristik des ersten physiologischen Signals umfasst:
Bestimmen eines dynamischen Bereichs von jedem der Vielzahl von Unterintervallen; und Bestimmen des dynamischen Bereichs des ersten physiologischen Signals basierend auf dem dynamischen Bereich von jedem der Vielzahl von Unterintervallen.

4. Das Verfahren nach Anspruch 3, wobei Bestimmen (304) der Amplitudenbereichscharakteristik des ersten physiologischen Signals basierend auf dem dynamischen Bereich von jedem der Vielzahl von Unterintervallen Bestimmen eines arithmetischen Mittels, eines Modus oder eines Medians des dynamischen Bereichs von jedem der Vielzahl von Unterintervallen umfasst.

5. Das Verfahren nach einem der vorhergehenden Ansprüche, ferner umfassend:
Skalieren des ersten physiologischen Signals basierend auf einem vorbestimmten Skalierungsfaktor; und
Anzeigen des skalierten ersten physiologischen Signals auf der Anzeige.

6. Das Verfahren nach Anspruch 5, wobei der vorbestimmte Skalierungsfaktor umfasst:
einen Skalierungsfaktor, der aus einer oder mehreren vorherigen Sitzungen bestimmt wurde, oder einen Skalierungsfaktor, der während Initialisierung bestimmt wurde.

7. Das Verfahren nach einem der vorhergehenden Ansprüche, ferner umfassend:
Messen eines dritten physiologischen Signals unter Verwendung des physiologischen Sensors während eines dritten Zeitintervalls;
Bestimmen einer Amplitudenbereichscharakteristik des zweiten physiologischen Signals;
gemäß einer Bestimmung, dass die Amplitudenbereichscharakteristik des zweiten physiologischen Signals das eine oder die mehreren Kriterien erfüllt:
Bestimmen eines zweiten Skalierungsfaktors, der sich von dem ersten Skalierungsfaktor unterscheidet, gemäß zumindest der Amplitudenbereichscharakteristik des zweiten physiologischen Signals;
Skalieren des dritten physiologischen Signals basierend auf dem bestimmten zweiten Skalierungsfaktor; und
Anzeigen des skalierten dritten physiologischen Signals auf der Anzeige.

8. Das Verfahren nach Anspruch 7, wobei:
Bestimmen des zweiten Skalierungsfaktors gemäß zumindest der Amplitudenbereichscharakteristik des zweiten physiologischen Signals Bestimmen des zweiten Skalierungsfaktors basierend auf einem arithmetischen Mittel, Median oder Modus der Amplitudenbereichscharakteristik des zweiten physiologischen Signals und der Amplitudenbereichscharakteristik des ersten physiologischen Signals umfasst.

9. Das Verfahren nach einem der vorhergehenden Ansprüche, wobei das erste Intervall und das zweite Intervall
aufeinanderfolgende Zeitperioden sind.

10. Eine elektronische Vorrichtung, umfassend:
einen physiologischen Sensor (140, 160, 202); und
eine oder mehrere Verarbeitungsschaltungen, die mit dem physiologischen Sensor gekoppelt sind, wobei die eine oder die mehreren Verarbeitungsschaltungen konfiguriert sind, um ein Verfahren nach einem der Ansprüche 1-9 durchzuführen.

11. Ein computerlesbares Medium, das Anweisungen umfasst, die, wenn sie von einer Vorrichtung ausgeführt werden, die einen physiologischen Sensor (140, 160, 202) und eine oder mehrere Verarbeitungsschaltungen umfasst, die eine oder die mehreren Verarbeitungsschaltungen veranlassen, ein Verfahren nach einem der Ansprüche 1-9 durchzuführen.

## Revendications

1. Un procédé comprenant :
la mesure (302) d'un premier signal physiologique à l'aide d'un capteur physiologique (140, 160, 202) pendant un premier intervalle de temps ;
la mesure (302) d'un second signal physiologique à l'aide due capteur physiologique pendant un second intervalle de temps, le premier signal physiologique et le second signal physiologique correspondant respectivement à un premier et à un second signal d'électrocardiogramme ;
la détermination (304) d'une caractéristique de plage d'amplitude du premier signal physiologique, la caractéristique de plage d'amplitude du premier signal physiologique étant une plage dynamique ;
suite à une détermination que la caractéristique de plage d'amplitude du premier signal physiologique vérifie un ou plusieurs critères, les un ou plusieurs critères comprenant un critère qui requiert que la caractéristique de plage d'amplitude du premier signal physiologique soit au-dessous d'un premier seuil de caractéristique de plage d'amplitude (923) :
la détermination (310) d'un premier facteur d'échelle en fonction de la caractéristique de plage d'amplitude du premier signal physiologique, la détermination du premier facteur d'échelle en fonction de la caractéristique de plage d'amplitude du premier signal physiologique comprenant :
suite à une détermination que la caractéristique de plage d'amplitude du premier signal physiologique est au-dessous d'un second seuil de caractéristique de plage d'amplitude (922), la détermination que le premier facteur d'échelle est un facteur d'échelle maximal prédéterminé ; et
suite à une détermination que la caractéristique de plage d'amplitude du premier signal physiologique est au-dessus du second seuil de caractéristique de plage d'amplitude (922) et au-dessous du premier seuil de caractéristique de plage d'amplitude (923), la détermination que le premier facteur d'échelle est entre le facteur d'échelle maximal prédéterminé et un facteur d'échelle minimal ;
la mise à l'échelle (312) du signal physiologique sur la base du premier facteur d'échelle déterminé ; et
l'affichage (314) du second signal physiologique mis à l'échelle sur un afficheur (400, 600, 800) ; et
suite à une détermination que la caractéristique de plage d'amplitude du premier signal physiologique ne vérifie pas les un ou plusieurs critères :
l'affichage du second signal physiologique sur l'afficheur.

2. Le procédé de la revendication 1, dans lequel la détermination (304) de la caractéristique de plage d'amplitude du premier signal physiologique comprend la détermination d'une différence entre une valeur d'amplitude maximale du premier signal physiologique pendant le premier intervalle de temps et une valeur d'amplitude minimale du premier signal physiologique pendant le premier intervalle de temps.

3. Le procédé de la revendication 1, dans lequel le premier intervalle de temps comprend une pluralité de sous-intervalles, et dans lequel la détermination (304) de la caractéristique de plage d'amplitude du premier signal physiologique comprend :
la détermination d'une plage dynamique de chacun de la pluralité de sous-intervalles ; et
la détermination de la plage dynamique du premier signal physiologique sur la base de la plage dynamique de chacun de la pluralité de sous-intervalles.

4. Le procédé de la revendication 3, dans lequel la détermination (304) de la caractéristique de plage d'amplitude du premier signal physiologique sur la base de la plage dynamique de chacun de la pluralité de sous-intervalles comprend la détermination d'une moyenne arithmétique, d'un mode, ou d'une médiane de la plage dynamique de chacun de la pluralité de sous-intervalles.

5. Le procédé de l'une des revendications précédentes, comprenant en outre :
la mise à l'échelle du premier signal physiologique sur la base d'un facteur d'échelle prédéterminé ; et
l'affichage du premier signal physiologique mis à l'échelle sur l'afficheur.

6. Le procédé de la revendication 5, dans lequel le facteur d'échelle prédéterminé comprend un facteur d'échelle déterminé lors d'une ou plusieurs sessions antérieures, ou un facteur d'échelle déterminé pendant une initialisation.

7. Le procédé de l'une des revendications précédentes, comprenant en outre :
la mesure d'un troisième signal physiologique à l'aide du capteur physiologique pendant un troisième intervalle de temps ;
la détermination d'une caractéristique de plage d'amplitude du second signal physiologique ;
suite à une détermination que la caractéristique de plage d'amplitude du second signal physiologique vérifie les un ou plusieurs critères :
la détermination d'un second facteur d'échelle, différent du premier facteur d'échelle, en fonction au moins de la caractéristique de plage d'amplitude du second signal physiologique ;
la mise à l'échelle du troisième signal physiologique sur la base du second facteur d'échelle déterminé ; et
l'affichage du troisième signal physiologique mis à l'échelle sur l'afficheur.

8. Le procédé de la revendication 7, dans lequel :
la détermination du second facteur d'échelle en fonction au moins de la caractéristique de plage d'amplitude du second signal physiologique comprend la détermination du second facteur d'échelle sur la base d'une moyenne arithmétique, d'une médiane, ou d'un mode de la caractéristique de plage d'amplitude du second signal physiologique et de la caractéristique de plage d'amplitude du premier signal physiologique.

9. Le procédé de l'une des revendications précédentes, dans lequel le premier intervalle et le second intervalle sont des périodes de temps consécutives.

10. Un dispositif électronique comprenant :
un capteur physiologique (140, 160, 202) ; et
un ou plusieurs circuits de traitement couplés au capteur physiologique, les un ou plusieurs circuits de traitement étant configurés pour mettre en œuvre un procédé selon l'une des revendications 1 à 9.

11. Un support lisible par calculateur comprenant des instructions qui, lorsqu'elles sont exécutées par un dispositif comprenant un capteur physiologique (140, 160, 202) et un ou plusieurs circuits de traitement, font en sorte que les un ou plusieurs circuits de traitement mette en œuvre un procédé selon l'une des revendications 1 à 9.
